# EUROPEAN PATENT APPLICATION

(11) **EP 4 287 212 A1**
(43) Date of publication of application: **06.12.2023**
(21) Application number: 22290036.7
(22) Date of filing: 30.05.2022
(51) Int. Cl.: G16H 50/50, G16H 50/70

(54) **MACHINE LEARNING PREDICTIVE MODELS OF TREATMENT RESPONSE**

(71) Applicant: Sophia Genetics S.A., 1025 Saint Sulpice (CH)
(72) Inventor: The designation of the inventor has not yet been filed
(74) Representative: Wenger, Joel-Théophile

(57) **Abstract**

The present invention is directed to a computer-implemented method of predicting treatment response or treatment efficacy of a patient based on the patient's multimodal features. In particular, the invention relates to methods for predicting lung cancer patients' response to immunotherapy treatment.

## Description

### Field

The present invention relates to the field of multimodal data integration and predictive models. In particular, the present invention relates to the computer-implemented methods of prediction of treatment response or treatment efficacy for a particular patient. In particular, the present invention relates to the computer-implemented methods of prediction of treatment response or treatment efficacy for cancer patients, such as lung cancer patients.

### Background

For many diseases there is more than one choice of treatment regimen that could be implemented and the choice for the specific patient is usually based on their clinal situation. Therefore, it would be of value to propose a predictive model of the clinical benefit associated with a specific treatment for a specific patient suffering for example from cancer (e.g., lung, breast or kidney cancer), neurological disorder or inherited disease (e.g., cardiological, neurological disease).

Triple negative breast cancer (TNBC) is a biologically and clinically heterogenous disease, associated with poorer outcomes when compared with other subtypes of breast cancer. Neoadjuvant chemotherapy (NCT) is often given prior to surgery and achieving pathological complete response (pCR) has been associated with improved long-term outcomes in terms of EFS (event-free survival) and OS (overall survival). There is thus high clinical interest in the ability to accurately predict non-pCR status using baseline data.

Surgery is the standard of care for localized kidney cancer. Diagnostic imaging plays a critical role in disease staging and informs the extent of surgical resection (partial or radical nephrectomy, extended resection). In clinical routine, up to 15% of the tumors initially assessed as T1-T2 on imaging is upgraded to pT3a status post-surgery, implying a higher risk of relapse. The ability to correctly predict pT3a status pre-surgery would inform the surgical approach. An individualized prediction of the risk of clinical T1 or T2 tumors to be upstaged to pT3a is thus of high surgical interest.

Lung cancer constitutes a major public health burden. In 2020, lung cancer was estimated to be responsible for ~2.2 million new cancer diagnoses worldwide and constituted the leading cause of cancer-related mortality with ~1.8 million lung cancer-related deaths (WHO, 2020). Among the different types of lung cancer, non-small cell lung cancer (NSCLC) is representing ~85% of total lung cancer cases. Within NSCLC, cases are further classified as adenocarcinoma, squamous cell carcinoma, and large cell carcinoma *(*Travis et al. 2015, J Thorac Oncol. 10(9):1243-1260*).*

First-line treatment of stage IV NSCLC requires systemic therapy. Historically, patients were treated with a doublet chemotherapy regimen, such as the combination of a platinum chemotherapy with gemcitabine, vinorelbine or a taxane. In this context, systemic therapy for NSCLC today is selected according to the presence of specific biomarkers. Strong oncogenic driver mutations have been identified in subsets of NSCLC patients in genes such as *EGFR, ALK, ROS1, BRAF* and *NTRK1*/*2*/*3.*

Collectively, these mutations account for approximately 30% of NSCLC cases, and render patients eligible for corresponding specific biomarker-driven targeted therapies. Given that patients eligible for such first-line targeted therapies currently only represent around 30% of all patients with stage IV NSCLC, the vast majority of NSCLC patients cannot benefit from this treatment approach *(*Arbour and Riely, 2019, JAMA. 322(8):764-774*).* For those patients, immunotherapy has dramatically altered the treatment landscape of stage IV NSCLC over the past few years.

Malignancies can overexpress PD-L1 as a mechanism of immune evasion, thereby downregulating the immune response towards the tumor through the inhibitory effects of the PD-L1/PD-1 interaction (programmed cell death (PD)-1 and anti-programmed cell death-ligand 1 (PD-L1)) *(*Pardoll et al., 2012, Nat Rev Cancer. 12(4):252-264*;* Dong et al., 2002, Nat Med. 8(8) 793-800*).* Antibodies directed against PD-1 or PD-L1 can block this interaction, resulting in "releasing the brakes" on the anti-tumor immune reaction. This therapeutic strategy has been successful across many tumor types, including NSCLC *(Pardoll et al, 2012, supra).*

In this context the immune checkpoint inhibitors anti-PD-(L)1 antibodies pembrolizumab, nivolumab and atezolimumab have been approved by the FDA and EMA as monotherapy regimens in the second-line NSCLC setting after progression on platinum-based chemotherapy *(*Herbst et al. 2016, Lancet. 387:1540-1550*;* Borghaei et al., 2015, NEJM, 373:1627-1639*;* Rittmeyer et al. 2017, Lancet 389:255-265). These second-line studies highlighted two key observations. First, while PD- L1 expression was shown to have some predictive power for response to immunotherapy using immune checkpoint inhibitors in some studies, the predictive value was not comparable to the targeted therapies in patients with specific genomic driver mutations. Second, lung adenocarcinoma patients harboring mutations in *EGFR* or *ALK* featured poor responses to immune checkpoint inhibitors compared to wild type tumors *(*Yang et al., 2020, Annu Rev Med, 71:117-136*).* In practice, metastatic NSCLC patients that do not have driver mutations amenable to targeted therapies nor contraindications to immunotherapy currently predominantly receive either pembrolizumab monotherapy when PD-L1 >50% or pembrolizumab plus chemotherapy combination therapy as first-line treatment. Increasingly, patients with PD-L1 >50% may also receive pembrolizumab plus chemotherapy combination therapy, although that practice is still evolving.

Despite the clinical promise of immunotherapy, significant challenges remain as the majority of NSCLC patients seem to be intrinsically resistant to immunotherapy and fail to respond. Overall, only approximately 20-30% of patients treated with immunotherapy show an objective response, although this varies by immune checkpoint inhibitor and clinical setting. At the same time patients are exposed to potentially severe side effects, especially immune-mediated reactions against healthy organs. Additionally, immune checkpoint inhibitors are particularly expensive, with most therapies costing in excess of USD 100'000 annually per patient, constituting a significant financial burden for healthcare systems.

Today, a high level of PD-L1 expression of 50% or above is the only standard predictive biomarker for immune checkpoint inhibitor efficacy as monotherapy in the first-line NSCLC setting *(*Remon et a/., 2020, J Thorac Oncol 15(6):914-947*).* PD-L1 however remains a suboptimal biomarker of immunotherapy response with several issues limiting its clinical utility. Differences in testing platforms, the use of various cut-off points for expression between different immunotherapy agents and the heterogenous nature PD-L1 expression within tumors have all been points of criticism *(*Bodor et al., 2020, Cancer 126:260-270*).* In this context the predictive power of PD-L1 for immunotherapy response remains limited. Indeed, even NSCLC patients with tumor PD-L1 >50% typically only display approximately 45% ORR, and patients with tumor PD-L1 >90% still only reach approximately 60% ORR *(*Aguilar et al., 2019, Ann Oncol. 30:1653-1659*).*

Some examples of a system and user interface were proposed to predict an expected response of a particular patient population when provided with a certain treatment (WO2020142551), however these are not directed to prediction of a specific patient biomarker signature and rather gives insight at the population level but not at the individual level.

In that context, it is of critical importance to validate approaches or biomarkers that can be predictive of the clinical benefit associated with a given treatment, such as with immune checkpoint inhibitors, in order to make the most effective, efficient and cost-effective use of these therapies. This could allow to offer the best available treatment to a specific patient based on his or her characteristics, while optimizing the resource allocation spend of healthcare systems.

Therefore, there is a universal need to compile and analyze the multimodal patient's data quickly, efficiently, and comprehensively.

### Summary

The present invention is based on the development of a particular computer-implemented method to predict treatment response or treatment efficacy for a specific patient based on the patient's at least two types of features selected from clinical, biological, genomic and radiological features, such as at least clinical and radiological features, and preferably consisting of clinical, biological, genomic and radiological features (wherein features of at least two types are referred herein as multimodal features). The computer-implemented methods of the invention use the patient's features that can be inputted to the machine learning algorithm, wherein said features are pre-processed raw patient's data selected from clinical, biological, genomic and radiological data. The computer-implemented methods of the invention use a combination of a trained imputation machine learning model trained to impute patient's missing features and a trained prediction machine learning model trained to predict patient's treatment response, as well as a list of informative features identifiers obtained during the prediction machine learning model training. It has been shown that the combination of machine learning models leads to increased accuracy of the method.

The methods of the invention are particularly suitable for processing of multimodal features or data received from real-world setting, wherein usually some data are missing, and data are containing a lot of noise. The methods of the invention are particularly suitable for using patients' clinical features or data, which on the one side are more easily accessible to be included in the prediction, but on the other side require more comprehensive analysis.

In one embodiment is provided a computer-implemented method of predicting treatment response of a patient, the method comprises steps of:
a) acquire
   i. a trained imputation machine learning model trained to impute patient's missing features,
   ii. a trained prediction machine learning model trained to predict patient's treatment response, and
   iii. a list of informative features identifiers used for the prediction machine learning model training wherein the trained imputation and prediction machine learning models were trained, and the list of informative features identifiers was obtained, using a set of multimodal features comprising at least two types of features selected from clinical, biological, genomic and radiological features of cohort of patients having the same disease and receiving the same treatment as the patient for whom the prediction is performed,
b) receive separately the patient's multimodal features comprising at least two types of features selected from clinical, biological, genomic and radiological features, wherein the patient's multimodal features are not complete,
c) aggregate the patient's multimodal features into a vector of features' values, wherein the vector of features' values is not complete,
d) input the vector of features' values to the trained imputation machine learning model and output a complete vector of features' values,
e) filter the features of the complete vector of features' values according to the list of informative features identifiers and obtain a predictive vector of features' values that is a subset of the complete vector of features' values consisting of filtered features' values,
f) input the predictive vector of features' values to the trained prediction machine learning model and output prediction of the patient's response to the treatment.

In another embodiment is provided the computer-implemented method of predicting treatment response of a patient according to the invention, wherein the prediction of the patient's response to the treatment is classified as a complete response, a partial response, a stable disease, or progression, or as a probability of the patient's response to the treatment.

In one embodiment is provided a computer-implemented method of predicting treatment efficacy of a patient, the method comprises steps of:
a) acquire
   i. a trained imputation machine learning model trained to impute patient's missing features,
   ii. a trained prediction machine learning model trained to predict patient's treatment efficacy, and
   iii. a list of informative features identifiers used for the prediction machine learning model training wherein the trained imputation and prediction machine learning models were trained, and the list of informative features identifiers was obtained, using a set of multimodal features comprising at least two types of features selected from clinical, biological, genomic and radiological features of cohort of patients having the same disease and receiving the same treatment as the patient for whom the prediction is performed,
b) receive separately the patient's multimodal features comprising at least two types of features selected from clinical, biological, genomic and radiological features, wherein the patient's multimodal features are not complete,
c) aggregate the patient's multimodal features into a vector of features' values, wherein the vector of features' values is not complete,
d) input the vector of features' values to the trained imputation machine learning model and output a complete vector of features' values,
e) filter the features of the complete vector of features' values according to the list of informative features identifiers and obtain a predictive vector of features' values that is a subset of the complete vector of features' values consisting of filtered features' values,
f) input the predictive vector of features' values to the trained prediction machine learning model and output prediction of the patient's treatment efficacy defined as length of time to an event.

In another embodiment is provided the computer-implemented method of predicting treatment efficacy of a patient according to the invention, wherein the patient's treatment efficacy is defined as length of time to an event and is selected from Progression-Free Survival (PFS), Overall Survival (OS), Duration of Response (DoR) and Time-To-Progression (TTP).

In another embodiment is provided the computer-implemented method of predicting treatment response of a patient according to the invention, or the computer-implemented method of predicting treatment efficacy of a patient according to the invention, wherein the output is complemented by a report with the list of informative features identifiers used for the prediction machine learning model training and/or a list of features' relative contributions used in the method of predicting treatment response or treatment efficacy of a patient.

In another embodiment is provided the computer-implemented method of predicting treatment response of a patient according to the invention, or the computer-implemented method of predicting treatment efficacy of a patient according to the invention, wherein in step d) features are imputed based on other feature modality.

In another embodiment is provided the computer-implemented method of predicting treatment response of a patient according to the invention,
wherein the prediction is made at baseline for a first evaluation time,
wherein the patient's multimodal features are collected at baseline, and
wherein the trained imputation and prediction machine learning models were trained, and the list of informative features identifiers was obtained, using multimodal features of cohort of patients having the same disease and receiving the same treatment as the patient for whom the prediction is performed, that were collected at baseline and using response at first evaluation time.

In another embodiment is provided the computer-implemented method of predicting treatment efficacy of a patient according to the invention,
wherein the prediction is made at baseline for a first evaluation time,
wherein the patient's multimodal features are collected at baseline, and
wherein the trained imputation and prediction machine learning models were trained, and the list of informative features identifiers was obtained, using multimodal features of cohort of patients having the same disease and receiving the same treatment as the patient for whom the prediction is performed, that were collected at baseline and using treatment efficacy result at first evaluation time.

In another embodiment is provided the computer-implemented method of predicting treatment response of a patient according to the invention, wherein the patient has cancer, and the treatment is immunotherapy, chemotherapy, a combination of immunotherapy and chemotherapy, neoadjuvant therapy, targeted therapy, surgery, radiation therapy, thermoablation, adjuvant therapy and/or hormonotherapy.

In another embodiment is provided the computer-implemented method of predicting treatment response of a patient according to the invention, wherein the patient has lung cancer, and the treatment is immunotherapy, chemotherapy, a combination of immunotherapy and chemotherapy, neoadjuvant therapy, targeted therapy, surgery, radiation therapy, thermoablation, and/or adjuvant therapy, and wherein the patient's multimodal features are comprising
- clinical features comprising a date of treatment initiation for the patient,
- biological features comprising a PD-L1 expression level at baseline,
- radiomics features comprising features extracted from the radiological imaging data at baseline,
- genomics features comprising the *EGFR* mutational status and the *ALK* mutational status at baseline. In another embodiment is provided the computer-implemented method of predicting treatment response of a patient according to the invention, or the computer-implemented method of predicting treatment efficacy of a patient according to the invention, wherein training of the imputation machine learning model comprises inputting to a machine learning supervised training algorithm a set of multimodal features comprising at least two types of features selected from clinical, biological, genomic and radiological features of cohort of patients having the same disease and receiving the same treatment as the patient for whom the prediction is performed, and wherein the trained imputation machine learning model produces as an output a complete list of features for a patient from an incomplete list.

In another embodiment is provided the computer-implemented method of predicting treatment response of a patient according to the invention, wherein a training of the prediction machine learning comprises inputting to a machine learning supervised training algorithm a set of multimodal features comprising at least two types of features selected from clinical, biological, genomic and radiological features of cohort of patients having the same disease and receiving the same treatment as the patient for whom the prediction is performed, and wherein the trained prediction machine learning model produces as an output a label classification of the patient's response to the treatment or a probability of the patient's response to the treatment, and a list of informative features identifiers used for the prediction machine learning model training.

In another embodiment is provided the computer-implemented method of predicting treatment efficacy of a patient according to the invention, wherein a training of the prediction machine learning comprises inputting to a machine learning supervised training algorithm a set of features comprising at least two types of features selected from clinical, biological, genomic and radiological features of cohort of patients having the same disease and receiving the same treatment as the patient for whom the prediction is performed, and wherein the trained prediction machine learning model produces as an output a label classification of the treatment efficacy defined as length of time to an event, and a list of informative features identifiers used for the prediction machine learning model training.

In another embodiment is provided the computer-implemented method of predicting treatment response of a patient according to the invention, or the computer-implemented method of predicting treatment efficacy of a patient according to the invention, wherein the patient's multimodal features are at least 75% complete.

### Description of the figures

**Figure** 1 shows selection of features for a patient for prediction of treatment response at first evaluation (t1).

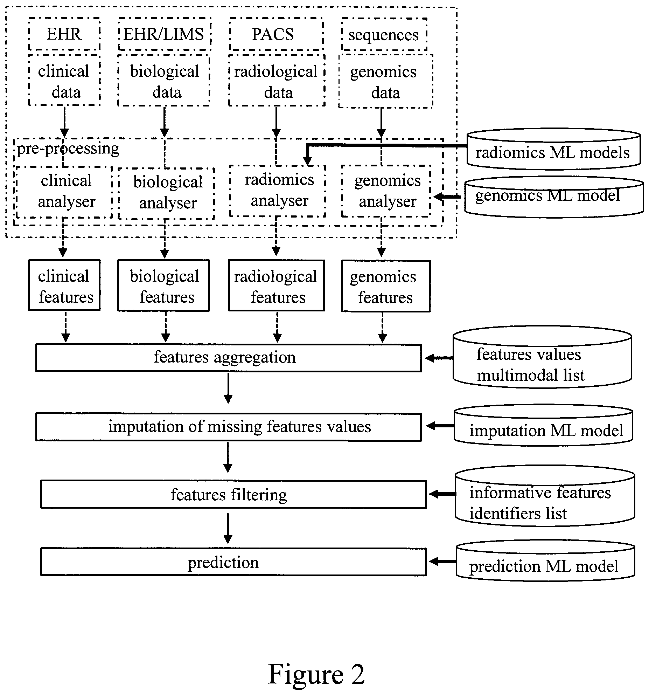

**Figure** 2 shows a processing system for use in a method to predict treatment response or treatment efficacy. HER - Electronic Health Record; PACS - Picture Archiving and Communication System;
LIMS - Laboratory Information Management System.

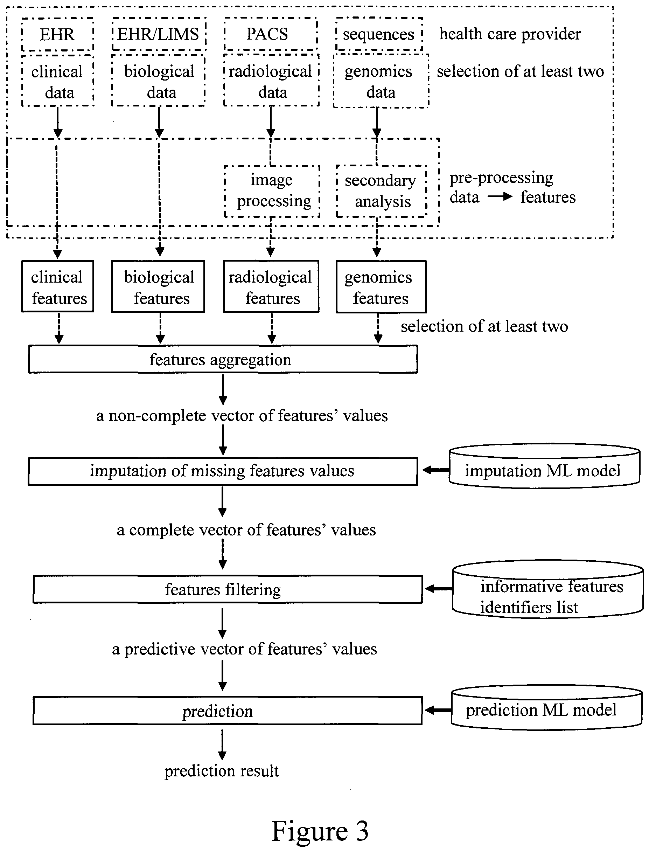

**Figure** 3 shows a processing workflow of a method to predict treatment response or treatment efficacy.

### Detailed description

The terms "baseline data", "baseline feature", "baseline clinical, biological, genomic and/or radiological data" or "baseline clinical, biological, genomic and/or radiological feature" refer to all data or features collected before treatment initiation. In that context "baseline" refers to a time before treatment initiation. It can be referred herein as time=0 or t0.

The terms "first evaluation (time) data", "first evaluation (time) feature", "first evaluation (time) clinical, biological, genomic and/or radiologicals data" or "first evaluation (time) clinical, biological, genomic and/or radiologicals feature" refer to all data or features collected after treatment initiation. In that context "first evaluation" refers to a time after treatment initiation. It can be referred herein as time=1 or t1. In one embodiment, first time evaluation is after treatment initiation, and is preferably about 2 to 4 months after the treatment initiation.

The terms "second/further evaluation (time) data", "second/further evaluation (time) feature", "second/further evaluation (time) clinical, biological, genomic and/or radiologicals data" or "second/further evaluation (time) clinical, biological, genomic and/or radiologicals feature" refer to all data or features collected after treatment continuation. In that context a second or further time evaluation is after treatment continuation and is preferably about 2 to 4 months after the treatment continuation (e.g., second/further dose). It can be referred herein as second evaluation time i.e., time=2 or t2, and as second evaluation time i.e., time=3 or t3 respective. In one embodiment, a second or further time evaluation is after treatment continuation and is preferably about 2 to 4 months after the treatment continuation (e.g., second/further dose).

It is understood that a "treatment" or a "therapy" refers to any treatment, such as cancer treatment. The "first-line treatment" or "primary/initial treatment" or "induction therapy" refers to the initial, or first treatment recommended for a given disease, such as cancer. For example, first-line treatment of stage IV NSCLC may be a pembrolizumab monotherapy, a chemotherapy and pembrolizumab combination therapy, a chemotherapy doublet and any other suitable treatment. "Second-line treatment" is treatment for a given disease, such as cancer after the first-line treatment has failed, stopped working, or has side effects that aren't tolerated.

It is understood that the patient's response to a treatment may be binary classified as (1) response (a complete or partial response) or (2) no response (a stable disease or progression). Alternatively, the patient's response to a treatment may be classified as (1) a complete response, wherein all the symptoms disappear and there is no evidence of disease; (2) a partial response, wherein the symptoms declined by a percentage, but disease remains; (3) a stable disease wherein the symptoms and the disease don't progress but are not decreasing or (4) progression, wherein the disease has further developed. It is understood that the patient's response to a cancer treatment may be classified as (1) a complete response, wherein all of the cancer or tumor disappears and there is no evidence of disease; (2) a partial response, wherein the cancer has shrunk by a percentage but disease remains; (3) a stable disease, wherein the cancer is neither shrinking nor growing (no change in cancer progression) or (4) progression, wherein there is a progression so that a cancer has further developed. Alternatively, the patient's response to a treatment may be provided as a probability of the patient's response to the treatment. In addition, confidence ranges may be provided.

The term "Progression-Free Survival (PFS)" refers to the length of time during and after the treatment of cancer, that a patient lives with the disease but it does not progress (assessed as tumour progression, the appearance of new lesions, and/or death).

The term "Overall Survival (OS)" refers to the length of time which begins at diagnosis (or at the start of treatment) and up to the time of death. The PFS and OS are commonly referred to as survival endpoints and measure the efficacy of cancer treatments.

The term "Duration of Response (DoR)" refers to the length of time from response (R) of cancer to a treatment (improvement) to the disease worsening again (progression/death). The DoR is commonly referred to as early efficacy endpoint.

The term "Time-To-Progression (TTP)" refers to the length of time from the date of diagnosis or the start of treatment for a cancer until the cancer starts to get worse or spread to other parts of the body. It is understood that PFS, OS, DoR and TTP are known endpoints for cancer clinical trials that are time-to-event data and measure the efficacy of cancer treatments.

In general, it is understood that "data" refers to information directly collected from patients' medical examination such as medical history, images, blood sample analysis, genomics test giving a list of variants, and the like. "Pre-processing" refers to a set of digital operation leading to the transformation of raw data that are directly collected from patients' medical examination to a set of "features" that can be used by the machine learning algorithm.

It is understood that one input to a machine learning algorithms variables (X) and output variables (Y). It is understood that imputation is the process of replacing missing data with substituted values. Thus, in the imputation process missing values are imputed. The term "imputation" refers to inferring, with a statistical analysis model, the value of missing data from available data in an incomplete data set, for instance when the data record has only been partially filled and/or some data elements or data features could not be calculated.

The term "extraction" refers to the signal processing analysis and/or calculation of a quantifiable value, such as a feature, from digital data, such as digital data file record, a digital health database, or a digital signal data input, such as for instance, but not limited to: an image or one or more image segments or elements, or a genomic sequence file or a genomic variant file, or a clinical annotation file, or a biological laboratory report file. Extraction may comprise various processing steps, such as for instance but not limited to: conversion, for instance to a predetermined unit; normalization, for instance to a scalar value in a range between 0 and 1; mathematical operations, for instance adding or multiplying or subtracting or dividing or deriving a value from one or more elements in the digital data to measure a property of the data, for instance the growth of an area or a volume; filtering part or all of the digital data signal, such as cropping, segmenting, extracting subsets such as patterns, regions or volumes of interest, removing elements, such as redundant information; transforming the digital data signal into a different representation space (e.g. from spatial domain to transform domain); digital signal processing analysis methods, or a combination thereof.

The term "aggregation" refers to combining, for instance by concatenation, multiple data inputs into a single data representation.

The term "selection" refers to identifying a subset of data elements into a data set.

The term "prediction" refers to inferring, with a statistical analysis model, the value of an outcome at a future time from the values of a data set at a current time.

A "machine learning model" refers to a data model or a data classifier which has been trained using a supervised, semi-supervised or unsupervised learning technique as known in the data science art, as opposed to an explicit statistical model. The data input may be represented as a 1D signal (vector), a 2D signal (matrix), or more generally a multidimensional array signal (for instance a tensor, or a RGB color image represented as 3^{∗}2D signals of its Red, Green and Blue color decomposition planes - 3 matrices), and/or a combination thereof. A multidimensional array is mathematically defined by a data structure arranged along at least two dimensions, each dimension recording more than 1 value.

In the case of a deep learning classifier, the data input is further processed through a series of data processing layers to implicitly capture the hidden data structures, the data signatures and underlying patterns. Thanks to the use of multiple data processing layers, deep learning facilitates the generalization of automated data processing to a diversity of complex pattern detection and data analysis tasks. The machine learning model may be trained within a supervised, semi-supervised or unsupervised learning framework. Within a supervised learning framework, a model learns a function to map an output result from an input data set, based on example pairs of inputs and matching outputs. Examples of machine learning models used for supervised learning include Support Vector Machines (SVM), regression analysis, linear regression, logistic regression, naive Bayes, linear discriminant analysis, decision trees, k-nearest neighbor algorithms, random forest, artificial neural networks (ANN) such as convolutional neural networks (CNN), recurrent neural networks (RNN), fully-connected neural networks, long short-term memory memory (LSTM) models, and others; and/or a combination thereof. A model trained within an unsupervised learning framework infers a function that identifies the hidden structure of a data set, without requiring prior knowledge on the data. Examples of unsupervised machine learning models known in the art include clustering such as k-means clustering, mixture model clustering, hierarchical clustering; anomaly detection methods; principal component analysis (PCA), independent component analysis (ICA), T-distributed Stochastic Neighbor Embedding (t-SNE); generative models; and/or unsupervised neural networks; autoencoders; and/or a combination thereof. Semi-supervised learning (SSL) is a machine learning framework within which one can train a model using both labeled and unlabeled data. Data augmentation methods can be optionally used to produce artificial data samples out of a scarce set of real data samples and increase the number and diversity of data used for model training. Unlabeled data, when used in conjunction with a small amount of labeled data, can produce considerable improvement in learning accuracy compared to other frameworks. This approach is particularly interesting when only part of the available data is labeled.

A "convolutional neural network" or "CNN" refers to a machine learning model which uses multiple data processing layers, known as convolutional layers, to represent the input data in a way which is best suited to solve a classification or regression task. During training, weight parameters are optimized for each CNN layer using optimization algorithms known in the art such as the backpropagation algorithm to perform a stochastic gradient descent. At runtime, the resulting trained CNN may then process very efficiently the input data, for instance to classify it into the right data output labels with as little false positives and false negatives as possible in the case of a learnt classification task. Convolutional neural networks may also be combined with recurrent neural networks to produce a deep learning classifier.

### Clinical, biological, genomic and radiological data

The term "multimodal data" or "multiomics data" refers to a set of at least two types of data selected from clinical, biological, genomic and radiological data. Any combination of at least two types of data selected from clinical, biological, genomic and radiological data can be referred to as multimodal or multiomics data. The possible sets may thus comprise or consist of: clinical and biological data; clinical and genomic data; clinical and radiological data; biological and genomic data; biological and radiological data; genomic and radiological data; clinical, biological and genomic data; clinical, biological and radiological data; biological, genomic and radiological data; clinical, genomic and radiological data; and clinical, biological, genomic and radiological data.

In a preferred embodiment, all the methods of the invention use at least clinical data combined with at least one data selected from biological, genomic and radiological data.

In another preferred embodiment, all the methods of the invention use clinical, biological, genomic and radiological data.

It is understood that the selection of at least two types of data (selected from of clinical, biological, genomic and radiological data) for use in the methods of the invention is based on specific patient cohort and prediction objective(s) as well as the current available evidence regarding their association with, and potential predictive power for predicting response to a selected treatment.

In one specific embodiment, at least clinical and biological data are used in methods as described herein, wherein a computer-implemented method to predict treatment response or prognosis or efficacy is used for a patient with kidney cancer.

In one specific embodiment, at least clinical, biological, and radiological data are used in methods as described herein, wherein a computer-implemented method to predict treatment response or prognosis or efficacy is used for a patient with brain cancer.

In one specific embodiment, clinical, biological, genomic and radiological data are used in methods as described herein, wherein a computer-implemented method to predict treatment response or prognosis or efficacy is used for a patient with stage IV non-small cell lung cancer (NSCLC).

In one embodiment, the multimodal data may be obtained or generated from one or more sources. This may include health care providers e.g., hospitals, primary care units, third parties providing services for medical data analysis and storage. For example, clinical and biological data may be obtained from databases that may be known by the terms of an electronic medical records (EMRs), an electronic health record (EHRs), a personal health records (PHRs) or an electronic case report form (eCRF). Clinical data may be obtained from Laboratory Information Management System (LIMS). Radiological data may be obtained from Picture Archiving and Communication System (PACS). Genomic data may be obtained from any system that stores genetic sequences, such as obtained through NGS VCF or FastO file.

The category definition and exemplary content of clinical, biological, genomic and radiological data are described herein. Further, in particular data are defined based on the example of a lung cancer.

### Clinical data

Clinical data for any patient may include, but are not limited to the patient's:
- demographics such as gender, age (month and year of birth), ethnicity, height and weight;
- medical history such as smoking status, personal history of diseases, previous familial history of diseases;
- disease history such as date of start of disease, disease stage, performance status at diagnosis, treatment history, hospitalization and/or death and organs affected, performance status and clinical response at first evaluation, progression status, including date and site of progression, treatment status after progression and vital status at last available news.

Clinical data for the cancer patient may include, but are not limited to the patient's:
- demographics such as gender, age (month and year of birth), ethnicity, height and weight;
- medical history such as smoking status, personal history of autoimmune diseases, pre-existing disease, previous familial history of cancer and previous personal history of cancer;
- disease history such as date of cancer, cancer stage, cancer subtype (IVA, IVB), performance status at diagnosis, corticosteroids and antibiotics treatment history, therapy received and number of cycles by progression and/or in total, presence of treatment toxicity leading to treatment discontinuation, hospitalization and/or death and organs affected, performance status and clinical response at first evaluation, progression status, including date and site of progression, treatment status after progression and vital status at last available news.

Clinical data for the patient with stage IV NSCLC diagnosis may include, but are not limited to the patient's:
- demographics such as gender, age (month and year of birth), ethnicity, height and weight;
- medical history such as smoking status, personal history of autoimmune diseases, pre-existing lung disease, previous familial history of cancer and previous personal history of cancer;
- disease history such as date of stage IV NSCLC diagnosis and subtype (IVA, IVB), performance status at stage IV NSCLC diagnosis, corticosteroids and antibiotics treatment history, therapy received and number of cycles by progression and/or in total, presence of treatment toxicity leading to treatment discontinuation, hospitalization and/or death and organs affected, performance status and clinical response at first evaluation, progression status, including date and site of progression, treatment status after progression and vital status at last available news.

In one embodiment, the clinical data for a patient with a lung cancer, in particular with stage IV NSCLC, may comprise at least one data on patient' age, Eastern Cooperative Oncology Group (ECOG) performance status (PS), autoimmune diseases history, steroid treatment history, gut microbiome status, antibiotics treatment history, disease history (such as liver metastasis, brain metastasis and bone metastasis) and immune-related adverse events.

In one embodiment, the clinical data for a patient with a lung cancer, in particular with stage IV NSCLC, may comprise at least one or consist of the following: gender, age (month and year of birth), ethnicity, height, weight, smoking status, personal history of autoimmune diseases, pre-existing lung disease, previous familial history of cancer, previous personal history of cancer, date of first NSCLC diagnosis, stage of first NSCLC diagnosis, date of stage IV NSCLC diagnosis, stage IV subtype at diagnosis (IVA or IVB), performance status at diagnosis, corticosteroids treatment received less than 12 months before stage IV NSCLC diagnosis, antibiotics treatment received less than 1 month before stage IV NSCLC diagnosis, start date of treatment with pembrolizumab, pembrolizumab and chemotherapy combination, or chemotherapy doublet, pembrolizumab dosing scheme, chemotherapy doublet regimen, number of cycles of therapy received by first evaluation, number of cycles of therapy received by progression, presence of treatment toxicity leading to treatment discontinuation, hospitalization, or death and organs affected, performance status at first evaluation, clinical response at first evaluation, progression status at last available update, date and site of progression, treatment status after progression, second-line therapy received, date of and viral status at last update, cause of death.

Clinical data refers to information that may comprise descriptive data such as patients' response to the treatment status, patient's disease progression. The descriptive data may be further categorized such as for example, the patient's response to the treatment may be classified as a complete response, a partial response, a stable disease or a progression, wherein the patient's disease progression may be classified as an increased growth speed and invasiveness of the tumor cells. This classification may be assigned numerical variables at the pre-processing step.

### Biological data

Biological data for the patient may include, but are not limited to the patient's: disease type and stage, expression level of relevant receptors, blood analysis at baseline and first/further evaluation (hematology and biochemistry).

Biological data for the cancer patient may include, but are not limited to the patient's: cancer stage IV and histopathology type at diagnosis, expression level of relevant receptors, blood analysis at baseline and first/further evaluation (hematology and biochemistry)

Biological data for the patient with stage IV NSCLC diagnosis may include but are not limited to the patient's stage IV NSCLC histopathology type at diagnosis, PD-L1 expression level, immunohistochemistry antibody used for PD-L1 measurement, blood analysis at baseline and first/further evaluation (hematology and biochemistry).

In one embodiment, the biological data for a patient with a lung cancer, in particular with stage IV NSCLC, may comprise data on PD-L1 expression on tumor cells.

In one embodiment, the biological data for a patient with a lung cancer, in particular with stage IV NSCLC, may comprise at least one data on neutrophil-to-lymphocyte ratio, enzyme lactate dehydrogenase (LDH) levels and/or blood TMB (bTMB).

In one embodiment, the biological data for a patient with a lung cancer, in particular with stage IV NSCLC, may comprise at least one or consist of the following: stage IV NSCLC histopathology at diagnosis, PD-L1 expression level, immunohistochemistry antibody used for PD-L1 measurement, date of blood analysis at baseline, white blood cells count at baseline, neutrophils count at baseline, lymphocytes count at baseline, monocytes count at baseline, eosinophils count at baseline, basophils count at baseline, platelets count at baseline, red blood cells count at baseline, hemoglobin levels at baseline, LDH levels at baseline, albumin levels at baseline, CRP levels at baseline, date of blood analysis at first evaluation, white blood cells count at first evaluation, neutrophils count at first evaluation, lymphocytes count at first evaluation, monocytes count at first evaluation, eosinophils count at first evaluation, basophils count at first evaluation, platelets count at first evaluation, red blood cells count at first evaluation, hemoglobin levels at first evaluation, LDH levels at first evaluation, albumin levels at first evaluation, CRP levels at first evaluation.

Biological data for the patient may include digital pathology data and proteomic data.

### Genomics data

The term "genomic data" refers to a digital representation of genomic information, such as a DNA sequence. In a next generation sequencing (NGS) bioinformatics workflow, genomic data may refer either to a raw nucleotide DNA sequence out from a sequencer (FASTQ file format), and/or to an aligned nucleotide sequence relative to a reference genome (BAM or SAM file format), and/or to a list of variants out from a variant calling step (VCF file format), and/or a list of annotated variants out of a variant annotation step (VCF file format).

A "variant" or a "genomic variant" refers to genomic sequence differences relative to a reference sequence. In bioinformatics data processing, a variant is uniquely identified by its position along a chromosome (chr,pos) and its difference relative to a reference genome at this position (ref, alt). Variants may include single nucleotide permutations (SNPs) or other single nucleotide variants (SNVs), insertions or deletions (INDELs), copy number variants (CNVs), as well as large rearrangements, substitutions, duplications, translocations, and others. In a bioinformatics secondary analysis workflow, a variant caller may apply variant calling to produce one or more variant calls listed in a Variant Calling File (VCF format). A germline variant is a variant inherited from at least one individual parent that differs from the wildtype genomic value as registered in a reference database, and that is present in all normal cells of the individual. A somatic variant or a somatic mutation or a somatic alteration is a variant caused by a genomic alteration, that is present in one or more somatic cells of the individual, for example in tumor cells.

A "mutation" or a "mutated gene" refers to a gene for each at least one variant has been identified. A "mutated gene status" may be classified as mutated in the latter case or normal otherwise. This status is routinely used as a biomarker in cancer diagnosis and prognosis. For instance the ALK gene mutation or the EGFR gene mutation have been shown of particular relevance in relation with lung cancer.

A "mutational load" or "mutation load" or "mutation burden" or "mutational burden", or for a tumor a "tumor mutational burden" or "tumor mutational load" or "TMB" refers to a biomarker measured as the number of somatic mutations per megabase of an interrogated genomic sequence.

A "MSI status" or "Microsatellite Instability status" or "Micro satellite instability status" refers to the status of a genomic alteration due to insertions or deletions of a few nucleotides in the microsatellite repeat regions based upon one nucleotide repeat (homopolymers) or a few nucleotides (heteropolymers), due a DNA mismatch repair system deficiency. This status is routinely used as a biomarker in cancer diagnosis and prognosis, and in particular in uterine, colon and stomach cancers such as UCES (Uterine Corpus Endometrial Carcinoma), COAD (Colon Adenocarcinoma) and STAD (Stomach adenocarcinoma). The MSI status of genomic alterations for a patient is usually categorized as:
- Micro-satellite stable, MSS: no evidence of any of the biomarker genomic loci exhibiting instability.
- Micro-satellite instable-low, MSI-L: evidence of instability in only one marker locus.
- Microsatellite instable-high, MSI-H: evidence of instability in at least two marker loci.

A "homologous recombination deficiency status" or "HRD status" refers to a classification of homologous recombination pathway and relates to any cellular state/event that results in homologous recombination pathway deficiency. HRD status may be classified as positive (HRD+) wherein a homologous recombination pathway is deficient or may be classified as negative (HRD-) wherein a homologous recombination pathway is not deficient or may be classified as undetermined otherwise (HRD uncertain, HRD unknown).

A "genomic pathway" or a "genetic pathway" refers a set of genomic loci or gene expression data which are significantly impacted in a given condition. In a bioinformatics secondary analysis workflow pathway analysis approaches use available pathway databases and the given genomic data or gene expression data from a patient to identify the presence or an absence of a genomic pathway for this patient.

Genomics data for the patient may include, but are not limited to the patient's disease site mutational status such as obtained through NGS VCF file. The patient's disease site may be a tumor or genetic material released by tumor and found in the blood.

Genomics data for the cancer patient may include but are not limited to the patient's cancer mutational status such as obtained through NGS VCF file.

In one embodiment, the genomics data for a patient with a lung cancer, in particular with stage IV NSCLC, may comprise at least one or consist of the following: *EGFR* and *ALK* mutational status. Tumor mutational status may be obtained through any known methods such as through NGS VCF file (based on locally available NGS panels), sanger sequencing, immunochemistry and the like.

In one embodiment, the genomics data for a patient with a lung cancer, in particular with stage IV NSCLC, may comprise data on tumor cell mutations such as at least one mutational status of *EGFR, ALK, KRAS, STK11*/*LKB1, KEAP1, PTEN, PIK3CA, TP53,* components of the DNA repair pathways such as including mismatch repair genes, *POLE* and *BRCA2,* components of the interferon-gamma (IFN-gamma) signaling such as including loss of function mutations in JAK1, JAK2 and beta-2-microglubulin (B2M).

In one embodiment, the genomics data for a patient with a lung cancer, in particular with stage IV NSCLC, may comprise data on tumor immunogenicity, such as tumor mutational burden (TMB), microsatellite instability (MSI) and defective mismatch repair (dMMR).

It is understood that genomics data may be collected only once at baseline or first or further evaluation or collected at multiple time points.

### Radiological/radiomics data

Radiological data, also referred to as radiomics data, are images collected and include, but are not limited to CT, PET, PET/CT MRI, SPECT, and the like.

Radiological data for the patient may include, but are not limited to the patient's imaging at pre-baseline, at baseline, at first/further evaluation.

The term "medical image data" or "radiological data" or "imaging data" refers to the digital images data and in particular one or more images collected for a patient at any time point during diagnosis and treatment. These images may be acquired from the patient examination in one or more medical centers in charge with one or more imaging modality such as CT, PET, MRI, SPECT, and others. These images may be in 2D or 3D format. These images may be securely collected, stored, archived and transmitted to the radiomics processing system of the invention in accordance with the PACS (Picture Archiving and Communication System) and DICOM (Digital Imaging and Communications in Medicine) digital medical imaging technology standards that are widely deployed in healthcare organizations worldwide. Radiological data for the cancer patient may include, but are not limited to the patient's: imaging at pre-baseline if available (millimetric injected CT cancer site scan at portal time, slices < 3 mm), imaging at baseline (millimetric injected CT cancer site scan at portal time, slices < 3 mm; PET/CT, CT, MRI if available), imaging at first evaluation (millimetric injected CT cancer site scan at portal time, slices < 3 mm; CT, MRI if available, imaging during follow-up visits after first evaluation if available, imaging at progression (millimetric injected CT cancer site scans at portal time, slices < 3 mm; CT, MRI if available), number of metastasis for each metastatic site at baseline and at first evaluation, RECIST (Response Evaluation Criteria in Solid Tumors) criteria if available.

In one embodiment, the radiological data for a patient with a lung cancer, in particular with stage IV NSCLC, may comprise data on imaging-based assessment of clinical tumor burden.

Radiological data for the patient with stage IV NSCLC diagnosis may include, but are not limited to the patient's: imaging at pre-baseline if available (millimetric injected CT thoracic, abdomen and pelvis scans at portal time, slices < 3 mm), imaging at baseline (millimetric injected CT thoracic, abdomen and pelvis scans at portal time, slices < 3 mm; PET/CT, brain CT, brain MRI if available), imaging at first evaluation (millimetric injected CT thoracic, abdomen and pelvis scans at portal time, slices < 3 mm; brain CT, brain MRI if available), imaging during follow-up visits after first evaluation if available, imaging at progression (millimetric injected CT thoracic, abdomen and pelvis scans at portal time, slices < 3 mm; brain CT, brain MRI if available), number of metastasis for each metastatic site at baseline and at first evaluation, RECIST criteria if available.

In one embodiment, the radiological data for a patient with a lung cancer, in particular with stage IV NSCLC, may comprise at least one or consist of the following: pre-baseline chest CT scan availability and date, baseline CT-TAP scan date, baseline CT-TAP RECIST (if available), baseline brain CT scan availability and date (if available), baseline PET/ CT scan availability and date (if available), baseline brain MRI availability and date (if available), extent of metastatic load assessment through imaging at baseline, extent of metastatic disease at baseline - status of affected organs, first evaluation chest CT scan date, first evaluation chest CT scan RECIST (if available), first evaluation CT-TAP scan availability and date (if available), first evaluation brain CT scan availability and date (if available), first evaluation brain MRI availability and date (if available), follow-up post-first evaluation chest CT scan date (if available), follow-up post-first evaluation chest CT scan RECIST (if available), follow-up post-first evaluation CT-TAP scan availability and date (if available), follow-up post-first evaluation brain CT scan availability and date (if available), progression evaluation chest CT scan date, progression evaluation chest CT scan RECIST (if available), progression evaluation CT-TAP scan availability and date (if available), progression evaluation brain CT scan availability and date (if available).

### Pre-processing and clinical, biological, genomic and radiological features

In one embodiment the obtained data are pre-processed, in a step that includes any known technique of data extraction and preparation so that they can be further inputted to and processed by a machine learning algorithm. It is understood that features refer to features values.

Pre-processing step may include steps such as recording data variables such as the categorical ones, computing gap times between events, performing descriptive statistics of data (minimum value, maximum, mean, median, etc. for numerical variables, count and frequencies for categorical variables) and the like.

Pre-processing step may include extracting data from patients' data platforms, such as SOPHiA DDM^{™} genomic module of the platform and/or SOPHiA DDM^{™} radiomics module of the platform. In this step extraction and harmonization of radiological images may be performed. In this step a secondary genomic analysis may be performed.

The term "multimodal features" or "multiomics features" refers to a set of at least two types of features selected from clinical, biological, genomic and radiological features. Any combination of at least two types of features selected from clinical, biological, genomic and radiological features can be referred to as multimodal or multiomics features. The possible sets may thus comprise or consist of: clinical and biological features; clinical and genomic features; clinical and radiological features; biological and genomic features; biological and radiological features; genomic and radiological features; clinical, biological and genomic features; clinical, biological and radiological features; biological, genomic and radiological features; clinical, genomic and radiological features; and clinical, biological, genomic and radiological features.

In a preferred embodiment, all the methods of the invention use at least clinical features combined with at least one feature type selected from biological, genomic and radiological features.

In another preferred embodiment, all the methods of the invention use clinical, biological, genomic and radiological features.

It is understood that the selection of at least two types of features (selected from of clinical, biological, genomic and radiological features) for use in methods of the invention is based on specific prediction objective(s) as well as the current available evidence regarding their association with, and potential predictive power for predicting response to a selected treatment.

In one specific embodiment, at least clinical and biological features are used in methods as described herein, wherein a computer-implemented method to predict treatment response or prognosis or efficacy is used for a patient with kidney cancer.

In one specific embodiment, at least clinical, biological, and radiological features are used in methods as described herein, wherein a computer-implemented method to predict treatment response or prognosis or efficacy is used for a patient with brain cancer.

In one specific embodiment, clinical, biological, genomic and radiological features are used in methods as described herein, wherein a computer-implemented method to predict treatment response or prognosis or efficacy is used for a patient with stage IV non-small cell lung cancer (NSCLC).

In one embodiment, the multimodal features may be obtained, acquired or generated from one or more sources. In another embodiment, the multimodal features may be obtained, acquired or generated separately from separate sources.

The set of clinical, biological, genomic and radiological features has a format that is compatible with machine learning models.

### Clinical features

In one embodiment, clinical data as described herein are pre-processed to obtain clinical features.

In one embodiment, clinical data refers to information that may comprise descriptive data such as patients' response to the treatment status, patient's disease progression, and the like. The descriptive data may be further categorized/classified and assigned numerical variables at the pre-processing step. In one embodiment, clinical data refers to information that may a date (e.g., date of start of treatment). The data may be transformed to a feature at the pre-processing step according to any known methods, e.g., a date may be transformed into a vector of several features values.

### Biologicalfeatures

In one embodiment, biological data as described herein are pre-processed to obtain biological features. In one embodiment, biological data refers to information that may comprise descriptive data such as patients' disease type and stage, expression level of relevant receptors, blood analysis, and the like. The descriptive data may be further categorized/classified and assigned numerical variables at the pre-processing step.

The data may be transformed to a feature at the pre-processing step according to any known methods, e.g., canonizing blood parameters (matching with reference) or construction of indicators like the neutrophile to lympcytes ratio and transforming into a vector of several features values.

### Genomics features

In one embodiment, genomics data as described herein are pre-processed to obtain genomics features. In one embodiment, genomic data may include the patient's disease state mutational status as described herein. The mutational status may be pre-processed to obtain a set of genomic features by any known methods.

It is understood that genomics features may be collected only once at baseline or first or further evaluation or collected at multiple time points.

In one embodiment, a pre-processing of genomics data may include a step of genomic secondary analysis or genomic tertiary analysis according to known methods with the use of known systems as described herein.

### Radiological features

In embodiment, the radiomic indicators or radiological features are extracted from of radiological images.

In one embodiment, radiological data are images as described herein, and said images are pre-processed to obtain a set of prepared radiological features according to known methods and systems.

The term "radiomics" as an abbreviation of "radiology omics" refers to the high-throughput digital extraction of mineable, quantitative data from radiological imaging data.

The term "radiomics feature", "radiomic indicator", "radiomic descriptor" or "imaging feature" refers to an imaging biomarker which can be extracted from imaging data as quantifiable summarization of the image, for instance a statistical value. The radiomics features or radiomics descriptors refer to a set of values computed from the segmentation of an image area (2D Region of Interest ROI or 3D Volume of Interest VOI), using the intensity values of the 2D pixels or 3D voxels included in the segmented ROI or VOI. In a radiomics workflow, multiple features, each representative of a different characteristic of the ROI or VOI in the image, may be individually extracted with a computer-implemented method and combined to produce a bank of features or a radiomics signature. This bank of features may include, but is not limited to morphological, heterogeneity and texture features. Example of banks of features commonly used in radiomics include any of 169 the well established features of the IBSI (image biomarker standardisation initiative) standard, any of the 1500 features of the Pyradiomics open source software package, and/or any of the LIFEx (www.lifexsoft.org), CERR, or IBEX public software tools.

### A method of predicting treatment response or treatment efficacy

In one embodiment, is provided a computer-implemented method of predicting treatment response or treatment efficacy of a patient based on the patient's multimodal features comprising at least two types of features selected from clinical, biological, genomic and radiological features.

Any combination of at least two types of features are selected from clinical, biological, genomic and radiological features can be used in the methods described herein and are referred to as multimodal features. The multimodal features can also be referred herein as multiomics features or a set of multimodal/ multiomics features.

In further embodiment, is provided a computer-implemented method of predicting treatment response or treatment efficacy of a patient based on the patient's multimodal data comprising acquiring at least two types of data selected from clinical, biological, genomic and radiological features. This computer-implemented method of predicting treatment response or treatment efficacy of a patient starting with patient's data thus comprise additional steps of:
- acquiring the patient's multimodal data comprising at least two types of data selected from clinical, biological, genomic and radiological data,
- pre-processing the acquired data and obtaining patient's multimodal features,
wherein the obtained features would be further processed as described herein for the computer-implemented method of predicting treatment response or treatment efficacy of a patient based on the patient's multimodal features.

It is thus understood that the selection of patients' data would be the same as selection of patients' features as described herein.

In a preferred embodiment, is provided a computer-implemented method of predicting treatment response or treatment efficacy of a patient that comprises acquiring at least patients' clinical features combined with at least one type of patients' features selected from biological, genomic and radiological features.

In another preferred embodiment, is provided a computer-implemented method of predicting treatment response or treatment efficacy of a patient that comprises acquiring at least patients' clinical and biological features.

In another preferred embodiment, is provided a computer-implemented method of predicting treatment response or treatment efficacy of a patient that comprises acquiring at least patients' clinical, biological, and radiological features.

In another preferred embodiment, is provided a computer-implemented method of predicting treatment response or treatment efficacy of a patient that comprises acquiring at least patients' clinical, biological, genomic and radiological data.

It is understood that independently whether the patient's multimodal features are defined as comprising any combination of at least two types of features selected from clinical, biological, genomic and radiological features, the steps of the computer-implemented method of the invention are performed in the same manner.

In one embodiment, is provided a computer-implemented method of predicting treatment response or treatment efficacy of a patient, the method comprising
- receiving the patient's multimodal features comprising at least two types of features selected from clinical, biological, genomic and radiological features
- inputting the patient's multimodal features to a first trained machine learning model, and inputting the output of the first trained machine learning model to another trained machine learning model, wherein the machine learning models have been trained to predict patient's treatment response or treatment efficacy using a set of features comprising at least two types of features selected from clinical, biological, genomic and radiological features of cohort of patients having the same disease and receiving the same treatment as the patient for whom the prediction is performed,
- predicting the patient's treatment response or treatment efficacy.

In one embodiment, is provided a computer-implemented method of predicting treatment response or treatment efficacy of a patient that uses at least two trained machine learning models. The models may be referred to as a first or second trained machine learning models depending on the sequence of their use in the methods of the invention.

In one embodiment, one machine learning model is trained to impute patient's missing features and may be referred to as an imputation machine learning model.

In another embodiment, another machine learning model is trained to predict patient's treatment response or treatment efficacy and may be referred to as a prediction machine learning model.

In another embodiment, the methods of the invention use a list of features identifiers that are identified during a training of a prediction machine learning model, and these may be referred to as informative features identifiers. Examples of identifiers include an index, a label, and the like. It is understood that selecting informative features identifiers is the common process when developing a predictive model and can be performed with any known methods.

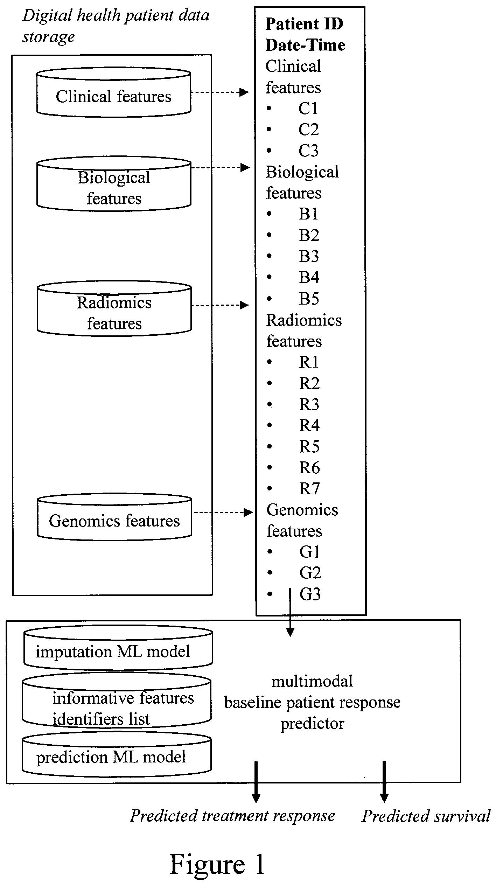

Figure 1 shows a list of informative features identifiers, wherein clinical informative features identifiers are exemplified as C1-3, biological informative features identifiers are exemplified as B1-B5, radiomics informative features identifiers are exemplified as R1-7 and genomics informative features identifiers are exemplified as G1-G3. It is understood that every informative feature identifier identifies a feature having a value.

In one embodiment, the trained machine learning model may be logistic regression, a Random Forest, a support-vector machine (SVM, also support-vector network), a Gradian Boosting method, MiceForest or any equivalent model. In one embodiment, the trained machine learning model is a supervised machine learning algorithm.

In one embodiment, is provided a machine learning model for imputing a patient's missing features for use in the computer-implemented method of predicting treatment response or treatment efficacy of a patient according to the invention, wherein training of the machine learning model is comprising inputting to a machine learning supervised training algorithm a set of features comprising at least two types of features selected from clinical, biological, genomic and radiological features of cohort of patients having the same disease and receiving the same treatment as the patient for whom the prediction is performed. The machine learning model for imputing a patient's missing features is trained to produce at its output a complete list of features for a patient from an incomplete list.

In another embodiment, is provided a machine learning model for prediction of patient's treatment response for use in the computer-implemented method of predicting treatment response of a patient according to the invention, wherein training of the machine learning model is comprising inputting to a machine learning supervised training algorithm a set of features comprising at least two types of features selected from clinical, biological, genomic and radiological features of cohort of patients having the same disease and receiving the same treatment as the patient for whom the prediction is performed. The machine learning model for predicting of patient's treatment response is trained to produce at its output a label classification of the patient's response to the treatment or a probability of the patient's response to the treatment and a list of informative features identifiers used for the prediction machine learning model training.

In another embodiment, is provided a machine learning model for prediction of patient's treatment efficacy for use in the computer-implemented method of predicting treatment efficacy of a patient according to the invention, wherein training of the machine learning model is comprising inputting to a machine learning supervised training algorithm a set of features comprising at least two types of features selected from clinical, biological, genomic and radiological features of cohort of patients having the same disease and receiving the same treatment as the patient for whom the prediction is performed. The machine learning model for predicting of patient's treatment efficacy is trained to produce at its output a label classification of the treatment efficacy defined as length of time to an event and a list of informative features identifiers used for the prediction machine learning model training.

In one embodiment, is provided a computer-implemented method of predicting treatment response or treatment efficacy of a patient based on the patient's multimodal features, wherein the features are acquired or received from external databases such as those that store pre-processed patient's data. In one embodiment, the patient's multimodal features are acquired or received separately.

In one embodiment, is provided a computer-implemented method of predicting treatment response or treatment efficacy of a patient based on the patient's multimodal features, wherein the features are not complete, in other words the acquired or received features are partial.

In one embodiment, the patient's multimodal features that are provided as input to a computer-implemented method of predicting patients' treatment response or treatment efficacy are not complete, and the method comprises a step of imputing missing features.

In one embodiment, the step of imputing missing features is performed when the patient's multimodal features are at least 60% complete, at least 65% complete, at least 70% complete, at least 75% complete, or preferably at least 75% complete.

In one embodiment, is provided a computer-implemented method of predicting treatment response or treatment efficacy of a patient based on the patient's multimodal features, wherein in one step the features are aggregated into a vector of features' values (also referred as a feature vector values). In one embodiment, the patient's multimodal features are not complete thus the features are aggregated into a vector of features' values that is not complete (a non-complete vector of features' values).

In one embodiment, is provided a computer-implemented method of predicting treatment response or treatment efficacy of a patient based on the patient's multimodal features, wherein in one step the non-complete vector of features' values is an input to the trained imputation machine learning model and an output is a complete vector of features' values. Therefore, missing features' values are imputed by the trained imputation machine learning model.

In one particular embodiment, features are imputed based on the same or different features modality. For example, *EGFR* mutation status may be imputed from radiomics features.

In one embodiment, is provided a computer-implemented method of predicting treatment response or treatment efficacy of a patient based on the patient's multimodal features, wherein in one step the complete vector of features' values is filtered according to the list of informative features identifiers obtained in the training of the prediction machine learning model. Therefore, a predictive vector of features' values that is a subset of the complete vector of features' values consisting of filtered features is obtained and may be referred herein as predictive complete feature vector, a predictive vector of features' values, a predictive feature vector values, a predictive complete feature vector or a predictive feature vector.

In one embodiment, is provided a computer-implemented method of predicting treatment response or treatment efficacy of a patient based on the patient's multimodal features, wherein in one step the predictive vector of features' values is an input to the trained prediction machine learning model and an output is prediction of the patient's response to the treatment or an output is prediction of the patient's treatment efficacy defined as length of time to an event.

In one embodiment, the prediction of the patient's response to the treatment is classified as a complete response, a partial response, a stable disease, or progression. In another embodiment, the prediction of the patient's response to the treatment is classified as a probability of the patient's response to the treatment. In another embodiment, the prediction of the patient's response to the treatment is binary classified as response or no response.

In another embodiment, the patient's treatment efficacy defined as length of time to an event is selected from selected from Progression-Free Survival (PFS), Overall Survival (OS), Duration of Response (DoR) and Time-To-Progression (TTP).

In another embodiment, is provided a computer-implemented method of predicting treatment response or treatment efficacy of a patient, wherein the output is complemented by a report with the list of informative features identifiers used for the prediction machine learning model training, or treatment features' relative contribution or weights used in the method of predicting treatment response or efficacy of a patient.

In another embodiment, is provided a computer-implemented method of predicting treatment response or treatment efficacy of a patient, wherein the prediction is made at baseline and the prediction of treatment response or treatment efficacy of a patient is for a first evaluation time. These computer-implemented methods of predicting treatment response or treatment efficacy of a patient use as input the patient's multimodal features collected at baseline. The computer-implemented methods of predicting treatment response of a patient uses the trained imputation machine learning model, the trained prediction machine learning model and the list of informative features identifiers, obtained using features of cohort of patients having the same disease and receiving the same treatment as the patient for whom the prediction is performed that were collected at baseline and using response at first evaluation time.

The computer-implemented method of predicting treatment efficacy of a patient use the trained imputation machine learning model, the trained prediction machine learning model and the list of informative features identifiers, obtained using features of cohort of patients having the same disease and receiving the same treatment as the patient for whom the prediction is performed that were collected at baseline and using treatment efficacy result at first evaluation time.

In other words, this computer-implemented method of predicting treatment response or efficacy of a patient use the trained imputation machine learning model, the trained prediction machine learning model and the list of informative features identifiers, obtained/trained using features of cohort of patients having the same disease and receiving the same treatment as the patient for whom the prediction is performed that were collected at baseline and wherein the treatment response or efficacy was known at first evaluation time.

In one embodiment, the selection of the patient's multimodal features that must be provided as an input to a computer-implemented method to predict the patient's treatment response or treatment efficacy is defined based on the specific patient condition.

In one embodiment, the set of the patient's multimodal data collected at baseline that must be pre-processed to features and provided as an input to a computer-implemented method of predicting treatment response of the patient with a lung cancer treated with immunotherapy, chemotherapy, or a combination thereof, and wherein the patient's multimodal data are comprising: the date of treatment initiation, PD-L1 expression level at baseline, imaging data at baseline, *EGFR* and *ALK* mutational status. Therefore, the patient's multimodal features must are comprising: clinical features comprising a date of treatment initiation for the patient, biological features comprising a PD-L1 expression level at baseline, radiomics features comprising features extracted from the radiological imaging data at baseline and genomics features comprising the *EGFR* mutational status and the *ALK* mutational status at baseline.

In another embodiment is provided the computer-implemented method of predicting treatment response of a patient according to the invention, or the computer-implemented method of predicting treatment efficacy of a patient according to the invention, wherein the patient has cancer, and the treatment is immunotherapy, chemotherapy, a combination of immunotherapy and chemotherapy, neoadjuvant therapy, targeted therapy, surgery, radiation therapy, thermoablation, adjuvant therapy and/or hormonotherapy.

In another embodiment is provided the computer-implemented method of predicting treatment response of a patient according to the invention, or the computer-implemented method of predicting treatment efficacy of a patient according to the invention, wherein the patient has lung cancer and the treatment is immunotherapy, chemotherapy, a combination of immunotherapy and chemotherapy, neoadjuvant therapy, targeted therapy, surgery, radiation therapy, thermoablation and/or adjuvant therapy, and wherein the patient's multimodal features are comprising clinical features comprising a date of treatment initiation for the patient, biological features comprising a PD-L1 expression level at baseline, radiomics features comprising features extracted from the radiological imaging data at baseline, genomics features comprising the *EGFR* mutational status and the *ALK* mutational status at baseline. In one embodiment, is provided a computer-implemented method of predicting treatment response or treatment efficacy to the second-line treatment with immunotherapy, such as anti-PD-1/PD-L1, chemotherapies, targeted therapies, PARP inhibitors, or combinations thereof, for patients with stage IV NSCLC treated in the first line setting with a pembrolizumab monotherapy, a chemotherapy and pembrolizumab combination therapy, or a chemotherapy doublet.

In one embodiment, is provided a computer-implemented method of predicting treatment efficacy for a patient, wherein the patient is predicted to completely or partially respond to the treatment, preferably based on described herein computer-implemented method of predicting treatment response of the patient.

In an alternative embodiment, is provided a computer-implemented method of predicting treatment response or treatment efficacy of a patient based on the patient's multimodal features, wherein the features are complete. It is understood that when a patient's multimodal features are complete, a computer-implemented method of predicting treatment response or treatment efficacy of a patient does not need to acquire a trained imputation machine learning model trained to impute patient's missing features and does not execute the step to input the non-complete vector of features' values to the trained imputation machine learning model and output a complete vector of features' values.

In one embodiment, is provided a computer-implemented method of predicting treatment response of a patient, the method comprises steps of:
a)acquire
   i. a trained prediction machine learning model trained to predict patient's treatment response, and
   ii. a list of informative features identifiers used for the prediction machine learning model training wherein the prediction machine learning model was trained, and the list of informative features identifiers was obtained, using a set of multimodal features comprising at least two types of features selected from clinical, biological, genomic and radiological features of cohort of patients having the same disease and receiving the same treatment as the patient for whom the prediction is performed,
b) receive separately the patient's multimodal features comprising at least two types of features selected from clinical, biological, genomic and radiological features, wherein the patient's multimodal features are complete,
c) aggregate the patient's multimodal features into a vector of features' values, wherein the vector of features' values is complete,
d) filter the features of the complete vector of features' values according to the list of informative features identifiers and obtain a predictive vector of features' values that is a subset of the complete vector of features' values consisting of filtered features' values,
f) input the predictive vector of features' values to the trained prediction machine learning model and output prediction of the patient's response to the treatment.

In one embodiment, is provided computer-implemented method of predicting treatment efficacy of a patient, the method comprises steps of:
a) acquire
   i. a trained prediction machine learning model trained to predict patient's treatment efficacy, and
   ii. a list of informative features identifiers used for the prediction machine learning model training wherein the prediction machine learning model was trained, and the list of informative features identifiers was obtained, using a set of multimodal features comprising at least two types of features selected from clinical, biological, genomic and radiological features of cohort of patients having the same disease and receiving the same treatment as the patient for whom the prediction is performed,
b) receive separately the patient's multimodal features comprising at least two types of features selected from clinical, biological, genomic and radiological features, wherein the patient's multimodal features are complete,
c) aggregate the patient's multimodal features into a vector of features' values, wherein the vector of features' values is complete,
d) filter the features of the complete vector of features' values according to the list of informative features identifiers and obtain a predictive vector of features' values that is a subset of the complete vector of features' values consisting of filtered features' values,
f) input the predictive vector of features' values to the trained prediction machine learning model and output prediction of the patient's treatment efficacy defined as length of time to an event.

In one embodiment, is provided a data processing apparatus comprising means for carrying out the method of predicting treatment response or treatment efficacy of a patient as described herein. In one embodiment, is provided a computer program comprising instructions which, when the program is executed by a computer, cause the computer to carry out the method of predicting treatment response or treatment efficacy of a patient as described herein.

In one embodiment, is provided a computer-readable medium comprising instructions which, when executed by a computer, cause the computer to carry out the method of predicting treatment response or treatment efficacy of a patient as described herein.

### Patients and treatments

In an embodiment, patients according to the invention are suffering from neurological disorders, a cancer, inherited cardiological or neurological disease, or rare inherited disease such as Pompe disease. In a particular embodiment, patients according to the invention are suffering from a cancer.

In another particular embodiment, patients according to the invention are suffering from cancer of brain, breast such as triple negative breast cancer (TNBC), kidney, head and neck, ovarian or colorectal cancer cancer.

In another particular embodiment, patients according to the invention are suffering from lung cancer, in particular non-small cell lung cancer (NSCLC).

In an embodiment, patients according to the invention are undergoing treatment for neurological disorders, inherited cardiological or neurological disease, or rare inherited disease such as Pompe disease.

In another particular embodiment, patients according to the invention are undergoing treatment for cancer, in particular immunotherapy, chemotherapy (such as neoadjuvant chemotherapy (NCT)), targeted therapy, treatment with anti-angiogenic drugs, surgery, radiation therapies or combinations of these treatments, and the like.

In another particular embodiment, patients according to the invention are undergoing treatment for cancer, in particular immunotherapy, chemotherapy, a combination of immunotherapy and chemotherapy, neoadjuvant therapy, targeted therapy, surgery, radiation therapy, thermoablation, adjuvant therapy and/or hormonotherapy.

In one embodiment, immunotherapy includes treatment with the immune checkpoint inhibitors anti-PD-(L)1 or anti-CTLA-4 antibodies Pembrolizumab, Nivolumab, Atezolimumab, Durvalumab, Cemiplimab, Dostarlimab.

In one embodiment patients are diagnosed with stage IV NSCLC *(de novo* or earlier stage progression to stage IV).

In another particular embodiment, patients are diagnosed with stage IV non-small cell lung cancer (NSCLC) with no actionable oncogene-driving mutations in *EGFR nor ALK in* first line of treatment. Patients with stage IV NSCLC may be treated in the first line setting with a pembrolizumab monotherapy, a chemotherapy and pembrolizumab combination therapy, or a chemotherapy doublet. In one embodiment patients are diagnosed with stage IV NSCLC without oncogene-activating mutations eligible for targeted therapy.

## Claims

1. A computer-implemented method of predicting treatment response of a patient, the method comprises steps of:
a) acquire
i. a trained imputation machine learning model trained to impute patient's missing features,
ii. a trained prediction machine learning model trained to predict patient's treatment response, and
iii. a list of informative features identifiers used for the prediction machine learning model training wherein the imputation and prediction machine learning models were trained, and the list of informative features identifiers was obtained, using a set of multimodal features comprising at least two types of features selected from clinical, biological, genomic and radiological features of cohort of patients having the same disease and receiving the same treatment as the patient for whom the prediction is performed,
b) receive separately the patient's multimodal features comprising at least two types of features selected from clinical, biological, genomic and radiological features, wherein the patient's multimodal features are not complete,
c) aggregate the patient's multimodal features into a vector of features' values, wherein the vector of features' values is not complete,
d) input the vector of features' values to the trained imputation machine learning model and output a complete vector of features' values,
e) filter the features of the complete vector of features' values according to the list of informative features identifiers and obtain a predictive vector of features' values that is a subset of the complete vector of features' values consisting of filtered features' values,
f) input the predictive vector of features' values to the trained prediction machine learning model and output prediction of the patient's response to the treatment.

2. The computer-implemented method of predicting treatment response of a patient according to claim 1,
wherein the prediction of the patient's response to the treatment is classified as a complete response, a partial response, a stable disease, or progression,
or as a probability of the patient's response to the treatment.

3. A computer-implemented method of predicting treatment efficacy of a patient, the method comprises steps of:
a) acquire
i. a trained imputation machine learning model trained to impute patient's missing features,
ii. a trained prediction machine learning model trained to predict patient's treatment efficacy, and
iii. a list of informative features identifiers used for the prediction machine learning model training wherein the imputation and prediction machine learning models were trained, and the list of informative features identifiers was obtained, using a set of multimodal features comprising at least two types of features selected from clinical, biological, genomic and radiological features of cohort of patients having the same disease and receiving the same treatment as the patient for whom the prediction is performed,
b) receive separately the patient's multimodal features comprising at least two types of features selected from clinical, biological, genomic and radiological features, wherein the patient's multimodal features are not complete,
c) aggregate the patient's multimodal features into a vector of features' values, wherein the vector of features' values is not complete,
d) input the vector of features' values to the trained imputation machine learning model and output a complete vector of features' values,
e) filter the features of the complete vector of features' values according to the list of informative features identifiers and obtain a predictive vector of features' values that is a subset of the complete vector of features' values consisting of filtered features' values,
f) input the predictive vector of features' values to the trained prediction machine learning model and output prediction of the patient's treatment efficacy defined as length of time to an event.

4. The computer-implemented method of predicting treatment efficacy of a patient according to claim 3,
wherein the patient's treatment efficacy is defined as length of time to an event and is selected from Progression-Free Survival (PFS), Overall Survival (OS), Duration of Response (DoR) and Time-To-Progression (TTP).

5. The computer-implemented method of predicting treatment response of a patient according to claim 1, or the computer-implemented method of predicting treatment efficacy of a patient according to claim 3,
wherein the output is complemented by a report with the list of informative features identifiers used for the prediction machine learning model training and/or a list of features' relative contributions used in the method of predicting treatment response or treatment efficacy of a patient.

6. The computer-implemented method of predicting treatment response of a patient according to claim 1, or the computer-implemented method of predicting treatment efficacy of a patient according to claim 3,
wherein in step d) features are imputed based on other feature modality.

7. The computer-implemented method of predicting treatment response of a patient according to any of claims 1, 2, 5 or 6,
wherein the prediction is made at baseline for a first evaluation time,
wherein the patient's multimodal features are collected at baseline, and
wherein the imputation and prediction machine learning models were trained, and the list of informative features identifiers was obtained, using multimodal features of cohort of patients having the same disease and receiving the same treatment as the patient for whom the prediction is performed, that were collected at baseline and using response at first evaluation time.

8. The computer-implemented method of predicting treatment efficacy of a patient according to any of claims 3, 4, 5 or 6,
wherein the prediction is made at baseline for a first evaluation time,
wherein the patient's multimodal features are collected at baseline, and
wherein the imputation and prediction machine learning models were trained, and the list of informative features identifiers was obtained, using multimodal features of cohort of patients having the same disease and receiving the same treatment as the patient for whom the prediction is performed, that were collected at baseline and using treatment efficacy result at first evaluation time.

9. The computer-implemented method of predicting treatment response of a patient according to any of claims 1, 2, 5 to 7,
wherein the patient has cancer, and the treatment is immunotherapy, chemotherapy, a combination of immunotherapy and chemotherapy, neoadjuvant therapy, targeted therapy, surgery, radiation therapy, thermoablation, adjuvant therapy and/or hormonotherapy.

10. The computer-implemented method of predicting treatment response of a patient according to claim 7,
wherein the patient has lung cancer and the treatment is immunotherapy, chemotherapy, a combination of immunotherapy and chemotherapy, neoadjuvant therapy, targeted therapy, surgery, radiation therapy, thermoablation and/or adjuvant therapy, and
wherein the patient's multimodal features are comprising
- clinical features comprising a date of treatment initiation for the patient,
- biological features comprising a PD-L1 expression level at baseline,
- radiomics features comprising features extracted from the radiological imaging data at baseline,
- genomics features comprising *EGFR* mutational status and *ALK* mutational status at baseline.

11. The computer-implemented method of predicting treatment response of a patient according to claim 1, or the computer-implemented method of predicting treatment efficacy of a patient according to claim 3,
wherein training of the imputation machine learning model comprises inputting to a machine learning supervised training algorithm a set of multimodal features comprising at least two types of features selected from clinical, biological, genomic and radiological features of cohort of patients having the same disease and receiving the same treatment as the patient for whom the prediction is performed, and wherein the trained imputation machine learning model produces as an output a complete list of features for a patient from an incomplete list.

12. The computer-implemented method of predicting treatment response of a patient according to claim 1,
wherein training of the prediction machine learning comprises inputting to a machine learning supervised training algorithm a set of multimodal features comprising at least two types of features selected from clinical, biological, genomic and radiological features of cohort of patients having the same disease and receiving the same treatment as the patient for whom the prediction is performed, and wherein the trained prediction machine learning model produces as an output a label classification of the patient's response to the treatment or a probability of the patient's response to the treatment, and a list of informative features identifiers used for the prediction machine learning model training.

13. The computer-implemented method of predicting treatment efficacy of a patient according to claim 3,
wherein training of the prediction machine learning comprises inputting to a machine learning supervised training algorithm a set of features comprising at least two types of features selected from clinical, biological, genomic and radiological features of cohort of patients having the same disease and receiving the same treatment as the patient for whom the prediction is performed,
and wherein the trained prediction machine learning model produces as an output a label classification of the treatment efficacy defined as length of time to an event, and
a list of informative features identifiers used for the prediction machine learning model training.

14. The computer-implemented method of predicting treatment response of a patient according to claim 1, or the computer-implemented method of predicting treatment efficacy of a patient according to claim 3,
wherein the patient's multimodal features are at least 75% complete.
